# EUROPEAN PATENT SPECIFICATION

(11) **EP 4 069 087 B1**
(45) Date of publication and mention of the grant of the patent: **30.04.2025**
(21) Application number: 20897435.2
(22) Date of filing: 26.11.2020
(51) Int. Cl.: A61N 5/10, A61B 6/00

(54) **COVER FOR A RADIOTHERAPY DEVICE**
ABDECKUNG FÜR EIN STRAHLENTHERAPIEGERÄT
COUVERCLE POUR UN DISPOSITIF DE RADIOTHÉRAPIE

(30) Priority: 06.12.2019 CN 201911244755
(43) Date of publication of application: 12.10.2022
(73) Proprietor: Elekta Beijing Medical Systems Co., Ltd, Beijing 102200 (CN)
(72) Inventor: JIANG, Zhongkui, Beijing 102200 (CN); ZHANG, Shijia, Beijing 102200 (CN)
(74) Representative: Collins, Emily Jane
(86) International application number: PCT/CN2020/131935
(87) International publication number: WO 2021/109920

(56) References cited:
- CN-A- 110 200 652
- CN-U- 206 183 288
- CN-U- 206 183 288
- CN-U- 208 942 179
- CN-U- 209 252 902
- CN-U- 209 529 160
- JP-A- 2017 213 321
- US-A1- 2018 289 981

## Description

### FIELD

The present disclosure relates to a cover for a radiotherapy device, and a radiotherapy device comprising a cover.

### BACKGROUND

Many radiotherapy apparatuses include a radiation source mounted on a gantry arm that is rotatable around a patient support on which a patient can be placed for treatment. The source is mounted at the end of the gantry arm, oriented so that the beam that it produces is directed towards the axis of rotation. The beam may be collimated by a collimator which is also mounted at the end of the gantry arm.

The point at which the centre of the beam meets the axis is known as the "isocentre". Thus, as the drum rotates, the beam arrives at the isocentre from all angular directions within a vertical plane. This allows a sufficient dose to be delivered to a target volume while minimising the dose delivered to surrounding healthy tissue.

The radiation head may need to be accessed by a maintenance engineer for, for example, maintenance or repair.

CN 206 183 288 teaches a system for a CT apparatus, the cover of which being held closed by default due to gravity or spring-operated forces.

### SUMMARY

Aspects and features of the present invention are described in the accompanying claims.

### BRIEF DESCRIPTION OF THE DRAWINGS

Specific embodiments are described below by way of example only and with reference to the accompanying drawings in which:
Figure 1 illustrates a radiotherapy apparatus;
Figure 2 illustrates a side view of a radiotherapy apparatus with the cover closed according to the present disclosure;
Figure 3 illustrates the radiotherapy apparatus with the gantry set to 0° and the cover open;
Figures 4 illustrates the radiotherapy apparatus with the gantry set to 180° and the cover open;
Figures 5A to 5D illustrate example components for use in the radiotherapy apparatus of the present disclosure.

### OVERVIEW

In radiotherapy it is important to take steps to reduce patient discomfort to improve the patient's experience. It is also important to lower patient anxiety, since stress is damaging to patient health and may negatively affect the radiotherapy treatment. Seeing the radiation source and other components, such as the collimators, may be intimidating to the patient and cause the patient stress. Therefore, in device of the present disclosure a cover is attached to the arm to conceal the components in the radiation head.

Since the radiation head includes a lot of compact components, the head may need to be accessed for servicing, maintenance, replacement or repair by an engineer. When an engineer visits a treatment site to service, maintain, repair and/or replace components in the radiation head, the machine cannot be used for treatment. This is, therefore, machine 'downtime'.

To access the radiation head the engineer must remove the cover. Time spent removing the cover increases the downtime of the machine. Typically removing components on a radiotherapy device is time consuming since the components are fixedly attached. Removing components may require a lot of man power and/or specialist tools.

The easy-open cover in the present disclosure can be opened by a single user with little effort. A single maintenance engineer can open the cover quickly and freely work on the radiation head whilst the cover is held in the open position. The radiation head can be accessed easily and quickly, reducing machine downtime.

### SPECIFIC DESCRIPTION OF CERTAIN EXAMPLE EMBODIMENTS

Throughout the figures, like reference numerals are used to indicate like parts.

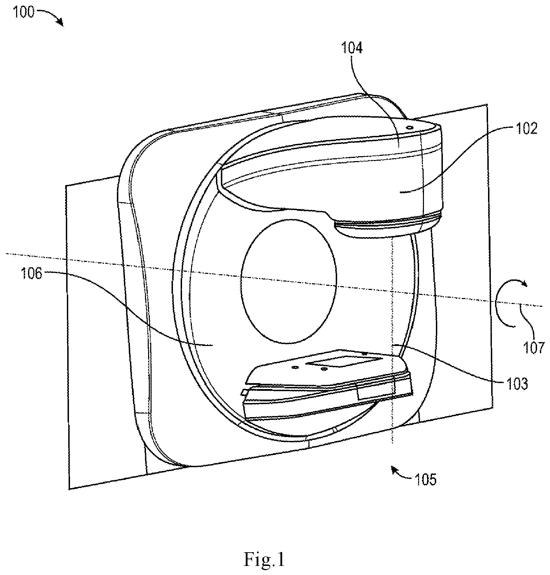

In Figure 1 a radiotherapy apparatus comprises a radiation head 102 comprising a source of radiation which emits a beam of radiation along a beam axis 105. The source is mounted on an arm 104 which is itself supported by a rotatable drum 106. The drum 106 may also be referred to as a gantry. The drum is arranged to rotate about a central axis 107 carrying with it the arm 104 and the source.

The arm 104 is mounted to the drum 106 so as to support the source at a location offset from the rotation axis 107 but pointing towards the rotation axis 107. The location at the meeting point of the beam axis 105 and the rotation axis 107 is referred to as the "isocentre". Thus, as the drum 106 rotates, the source rotates around the isocentre, directing a beam toward that isocentre continuously. The beam 105 can be delivered to the patient from all angles, allowing a sufficient dose to be delivered to a target volume while minimising the dose delivered to surrounding healthy tissue.

The arm 104 rotates though degrees of rotation along a rotation path. The arm at the top most point is defined as 0° of rotation, and the arm at the bottom most point is defined as 180° rotation.

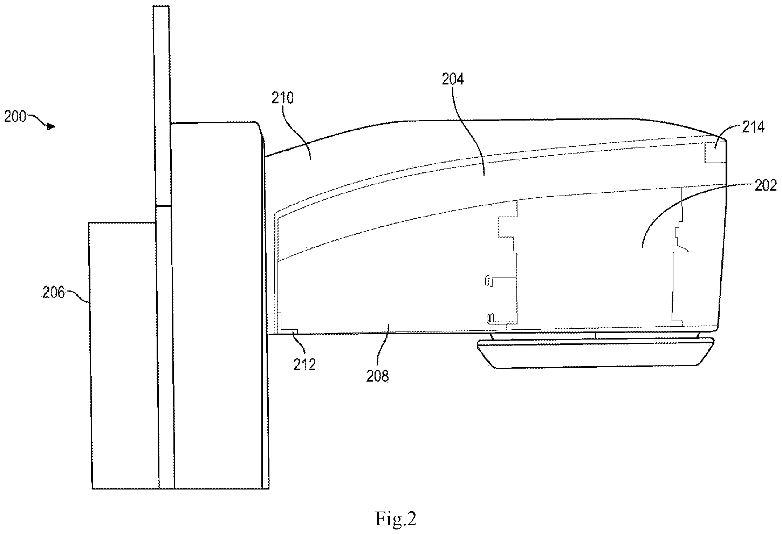

Figure 2 shows a side view of a radiotherapy apparatus 200 according to the present disclosure. The apparatus comprises a radiation head 202. The radiation head is configured to produce a beam of therapeutic or imaging radiation. The beam may be collimated (i.e. shaped) in accordance with a treatment plan. The radiation head may include a collimator, such as a multileaf collimator, for collimating the beam.

The radiation head is used to refer to the end of the arm which houses the radiation source, any collimation devices and any other components used for producing the radiation beam. The radiation source may be a linear accelerator, in which charged particles, such as electrons, are accelerated along a waveguide towards a target to produce therapeutic radiation. Housing is used to refer to the components of the radiotherapy apparatus which do not include the source of radiation or any collimation devices. That is, the housing is the structure, covers and associated features which provide the mechanism and support for a source of radiation to be used to deliver a beam of radiation according to a treatment plan.

The radiation head 202 is supported on an arm 204. The arm bears the weight of the radiation head and supports the radiation head. The arm 204 is attached to a drum 206. The drum 206 is configured to rotate around a central axis, carrying with it the arm 204. The rotation of the arm 204 causes corresponding rotation of the radiation head 202. In this way, radiation can be delivered to the patient from a plurality of angles. The drum may be able to rotate through 360°, or may be able to rotate through a smaller range, such as 270° or 180°. The arm has a fixed upper casing 210. This covers the outer side of the arm. The upper casing does not cover the radiation head 202.

The arm 204 and the drum 206 are a 'support structure' which support the radiation head and other components of the radiotherapy apparatus. The arm and the drum are fixed relative to one another.

The arm 204 has a near end, which is the end attached to the drum 206. The far end of the arm 204 is the end distal from, i.e. away from, the drum 206. Features are discussed throughout as being attached at or towards the near or far end. This language is used to refer to attachment towards the near or far end respectively. That is, it could refer to a point at the furthest end, alternatively could refer to a point near to the end. It could refer to a point anywhere in the half of the near/far end.

A cover 208 is attached to the arm 204. In Figure 2 the cover is shown in a closed position in which it conceals the radiation head 202. The cover may be made from glass reinforced plastic, GRP.

In the closed position the cover 208 forms a façade over the radiation head 202. That is, the cover blocks the radiation head from the view of the patient. The cover may cover all, or part, of the radiation head 202.

The cover is used to, as described above, conceal the components of the radiation head from the view of the patient. Additionally, the cover shields the components of the radiation head from the exterior environment. For example, the cover protects the source of radiation from dust or other particles in the treatment room. The cover therefore covers the components of the radiation head to conceal or shield the components from the view of the patient and to protect or shield the components from the exterior environment. The term shield is used to describe covering, or at least partially covering.

The arm 204 is positioned on the drum 206 radially outwards from the cover 208. That is, the cover 208 is positioned on the inside edge of the arm 204.

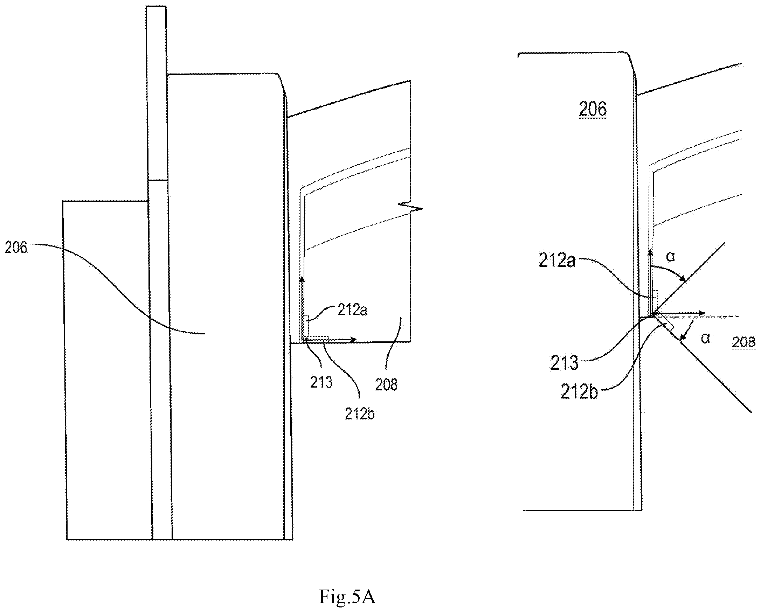

The cover is hingably attached to the support structure, for example the arm 204 or to the drum 206, at a first attachment point. The cover is attached to the drum or to the near end of the arm at the first attachment point by a hinge 212. An example of a hinge is discussed is more detail below in relation to Figure 5A. The first attachment point is on the support structure, meaning that the first attachment point is at a fixed location relative to the arm 204.

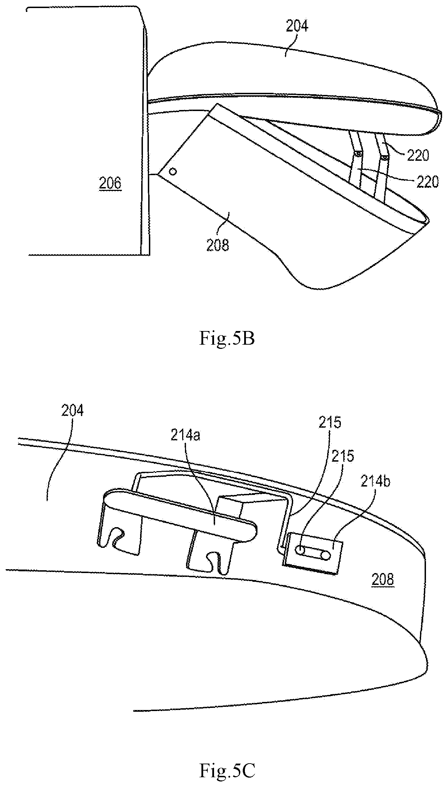

A locking mechanism 214 (or "lock" 214) connects the cover 208 to the arm 204 at a second attachment point. The locking mechanism is positioned at or near the distal end of the arm (i.e. the opposing end of the arm to the hinge 212). The locking mechanism 214 releasably attaches the cover 208 to the arm 204 at the second attachment point. When the locking mechanism is locked, the cover 204 is attached to the arm at two locations (i.e. the first and second attachment points) and therefore is held fixedly relative to the arm 204 in a position which conceals the radiation head 202. When the locking mechanism is released, the cover 208 is released from, and no longer attached to, the arm 204 at the second connection point. An example of a locking mechanism 214 is described in more detail below in Figure 5B.

In the locked state, in which the cover is closed, the connection is secure enough such that the cover 208 is secured to the arm 204 at all degrees of rotation of the arm 204 (i.e. the connection is secure enough to overcome the force of gravity). Further, the cover is held securely in the closed position and does not open upon rotation of the arm 204 around the centre axis, even when the arm 204 is rotated at relatively high speeds which are necessary during treatment. Therefore the cover will not open when the radiotherapy apparatus is in use, for example when a patient is being treated with therapeutic radiation. During treatment the cover is in the closed position and the components of the radiation head are shielded from view of the patient by the cover. This provides a less intimidating environment for the patient, which improves the patient's wellbeing.

The lock and hinge in the figures are illustrated as being positioned at the far end and the near end of the arm respectively. However, the lock and hinge may be positioned anywhere on the support structure to provide the necessary function. For example, the hinge could be positioned on the longitudinal side of the arm/cover such that the cover hinges open along the longitudinal side of the arm/cover. The lock mechanism may be positioned on the opposite longitudinal side of the arm/cover to the hinge.

In some examples the lock may be combined with the hinge, such that the hinge can be locked in the closed position, which in turn locks the cover in the closed position. That is, the hinge is fixable or lockable into a fixed position.

There may be times when the radiation head 202 needs to be accessed. This may be for maintenance, or servicing, such as testing to ensure all components are correctly functioning. When, for whatever reason, access to the radiation head is needed, the cover 208, which is in place to cover the components of the radiation head, prevents the engineer from directly accessing the radiation head. The cover must be moved or removed in order to provide access to the components in the radiation head.

As explained above, time spent removing the cover increases the downtime of the machine.

In the present apparatus, to access the radiation head 202 the cover 208 is moved from the closed position (in which the radiation head is concealed or covered by the cover) to the open position, in which the components of the radiation head are accessible to a user or operator. The cover 208 is a quick release cover. That is, the cover can be opened easily and without the need for tools or equipment. This is labour saving, and saves man power required to access the radiation head. Further, the radiation head can be accesses quickly, which reduced machine downtime for maintenance and repair.

Therefore, the cover 208 can be opened quickly.

In addition, the cover can be removed if necessary. To remove the cover 208, the hinge 212 is dismantled. In the device illustrated in the figures the hinge 212 is a hinge pin. To remove the cover completely, the pin is removed from between the hinged parts, allowing the hinged parts to be separated and the cover 208 to be completely removed from the arm 206.

In some aspects, the radiotherapy apparatus has a head touch guard ring and head ending cover 217. This ring covers the radiation head. The ring is positioned over the radiation head and external to the cover 206. In devices having the guard ring, the ring is removed before the cover 208 is opened.

The guard ring is a proximity sensor for the radiotherapy device. If an object contacts the guard ring, or comes within a set threshold distance of the guard ring, a signal is sent to a control system to initiate a shut-down of the radiotherapy device. The object could be a patient or component of the radiotherapy device. In shut-down the control system stops the radiotherapy device from moving or delivering treatment. Therefore the guard ring acts as a safety measure to mitigate the treatment arm colliding with a patent or other object.

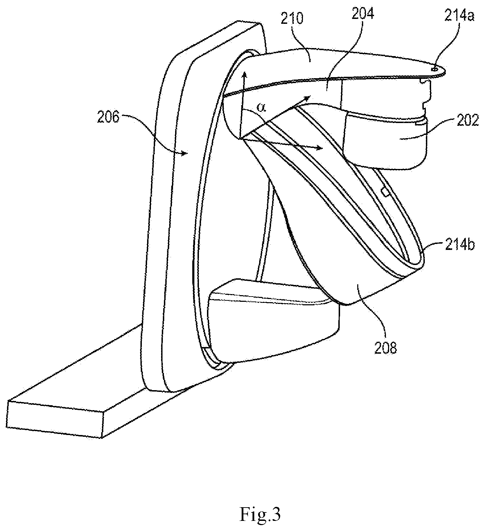

Figure 3 shows the radiotherapy apparatus of Figure 2 with the cover in the open position. The radiation head is in the 0° position, i.e. at its highest point and directed substantially vertically downwards.

The cover 208 is attached to the arm 204 and /or the drum 206 by the hinge 212. The hinge is in the open position such that the cover is angled relative to the arm (i.e. there is an angle between the cover 208 and the arm 204).

In the open position the cover 208 and the arm 204 are not connected at the second connection point. The cover has been moved downwards, i.e. away from the arm. The radiation head 202 which is shielded by the cover in the closed position, is exposed in the open position. Components of the radiation head are therefore accessible.

To move the cover from the closed position to the open position, the lock 214 is unlocked. The lock can be unlocked by a user, for example by a maintenance engineer. The lock 214 can be a quick release lock, meaning it can be unlocked with a single, or with a simple, application of pressure from the user.

In the lock shown in the figures, the lock 214 is includes locking parts 214a and 214b which are integral to, or fixedly attached on, the arm 204 and the cover 208 respectively. The lock 214 has a locked position in which the locking parts 214a and 214b are securely fastened together. Since the locking parts 214a and 214b are positioned on the arm 204 and the cover 208 respectively, when the lock is in the locked position the arm and the cover are held together securely. This is the closed position (shown in Figure 2) in which the radiation head 202 is concealed by the cover 208. In the open position the parts are not securely held together, such that the parts 214a and 214b may be separated and the cover 208 is not securely held to the arm 204 at the second connection point. The cover 208 is secured at the near end to the drum 206 or to the arm 204 by the hinge 212, and at the far end the cover 208 is not secured to the arm 204. With the radiation head in the 0° position, i.e. at its highest point and directed substantially vertically downwards, gravity acts to move the cover 208 away from the arm 204. With the radiation head 202 in the 0° position, the arm is at the top of the rotation path, and the cover 208 is positioned below the arm 204. Therefore the cover 208 can move away from the arm 204 due to the downwards force of gravity.

In the 0° position, gravity acts on the cover 208. The cover 208 is secured at the near end to the drum 206 or to the arm 204 by the hinge 212, but is not secured to the arm 204 at the far end with the lock 214 is in the unlocked position. Gravity acts on the cover 208 such that the cover rotates away from the arm 204 around the hinge 212, revealing the radiation head 202. In the open position the cover 208 is at an angle relative to the arm 204.

As the cover 208 opens, it rotates on hinge 212. The angle of rotation of the cover 208 about the hinge 212 increased as the cover 208 opens more widely, and as the far end of the cover 208 moves further from the far end of the arm 204. The 'angle' of the cover is used to describe the angle at which the cover rotates open relative to the arm. It is illustrated as α on the figures. In the closed potion, alpha is zero. In the open position, alpha could be, for example, 45° or 54°. With the cover 208 in the open position the radiation head 202 is accessible to a user. That is, cover 208 does not conceal the radiation 202 head when in the open position. The radiation head can therefore be accessed by a maintenance engineer for maintenance, service or repair.

When the user no longer needs to access the radiation head, for example when the radiation head has been repaired or serviced, the user can close the cover 208 by moving the far end of the cover towards the arm 204. When the far end of the cover 208 is fully closed and touches the arm 204, the lock 214 is locked to secure the cover 208 to the arm 214 at the far end to maintain the cover 208 in the closed position. The lock 214 may automatically lock (i.e. move to the locked position) upon contact of the far end of the cover 208 with the arm 204. Alternatively, the user may physically lock the lock 214 to secure the ends together. In this locked position the cover 208 is secured in the closed position and the radiotherapy apparatus can be used for treating patients.

In the above embodiment the cover 208 moves to the open position using the force of gravity. When the device is in the 0° position, with the radiation source at the top and pointing vertically downwards, the cover 208 is beneath the arm 204 and therefore can be hinged away from the arm using the force of gravity.

There may be occasions when access to the radiation head is required when the device is at the 180° position. There may be operations which require the device to be in the 180° position. Some operations (e.g. repair or maintenance) may be easier with the device in this position. In this position the arm 204 is at the bottom of the rotation path. The radiation source is therefore at the bottom and is pointing vertically upwards. In the 180° position, the cover 208 is above the arm 204. Therefore gravity acts to pull the cover down on to and towards the arm 204. In this position gravity therefore will not cause the cover 208 to move to the open position, even if the lock 214 is unlocked.

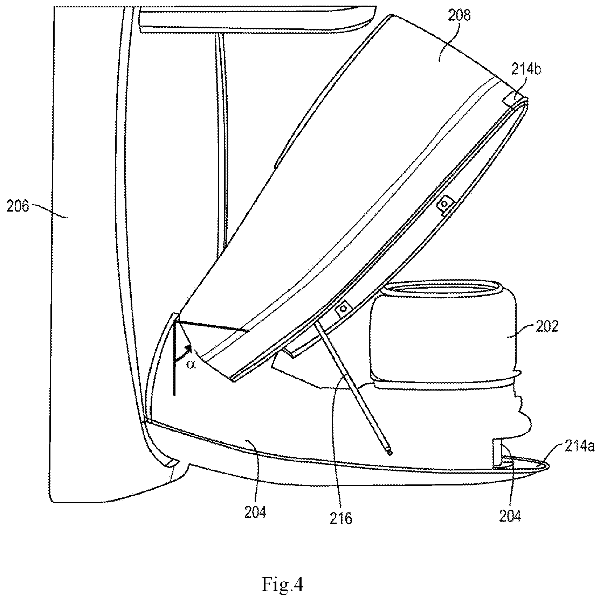

Figure 4 shows the radiotherapy apparatus 200 in the 180° position.

To provide an apparatus which allows a user to access the radiation head more easily and without the need for additional man power, an optional additional feature can be included in the radiotherapy apparatus. The features assist the user when pushing the cover 208 up and away from the arm 204 into the open position.

The apparatus can also include a spring mechanism 216. In the figures the spring mechanism is an air spring. However, any suitable spring could be used.

The spring 216 is fixedly attached at one end to the arm 206 and is fixedly attached at the opposing end to the arm 204. The spring can be positioned anywhere along the length of the cover. That is, it could be positioned anywhere between the hinge 212 and the far end of the cover. The spring mechanism 216 is configured to bias the cover 208 away from the arm 204 by providing a biasing force between the cover 208 and the arm 204.

The spring 216 provides an outwards biasing force between the arm 204 and the cover 208. That is, the spring biases the cover 208 towards the open position. If the lock 214 is in the unlocked state (that is, if the cover is not attached to the arm at the far end) the spring will then provide a force which will move the cover from the closed position to the open position. Depending on the strength of the spring, it may not exert enough force to move the cover to the open position, however it will exert a force biasing the cover 208 into the open position, so a user only needs to provide the additional force (the delta) to open the cover. That is, the spring makes it easier for the user to open the cover by reducing the force required by the user.

When a spring is compressed from its resting position, it exerts an opposing force. When a spring is at its resting position, it no longer exerts a force.

The spring 216 in Figures 2 to 5 provides a biasing force between the cover 208 and the arm 204 up to a certain angle of rotation of the cover 208 about the hinge 212 at which the spring has reached its resting position. The system uses a spring which has a resting position that corresponds to the cover 208 being open in the optimal position to allow the radiation head to be accessed. This optimal position is the open position. The spring 216 provides a biasing force up to an angle of rotation at which the cover 208 is in the open position, after which is does not provide further rotation.

The angle of rotation of the cover 208 about the hinge 212 is alpha in the figures, and is described as the open angle.

In the figures the spring 216 has a resting position which corresponds to an open angle of 54°. Therefore, when the cover 208 opens to an angle of 54°, the spring no longer exerts a biasing force in the opening direction. The cover 208 will stop opening once it has reached 54°. This angle is optimal to provide access to the radiation head whilst not being too far open that it becomes difficult to close the cover 208 again.

In other examples the resting position of the spring 216 may correspond to an open angle of between 53° and 55°. In other examples, the open angle is between 50 and 60°. In further examples the open angle is between 40 and 70°. Other open angles can be envisaged, and the optimal open angle may vary depending on the dimensions of the specific device.

In one example two springs 216 used, one spring positioned on each side of the arm 206. This provides a higher opening force to the cover. In other examples, there is a single spring 206 attached on only one side of the arm 206. In other examples still, there are no springs on the device.

In use, when the locking mechanism 214 is unlocked, the far end of the cover 208 is not attached to the far end of the arm 204. The spring mechanism 216 pushes the cover 208 away from the arm 204. The cover 208 rotates around the hinge 212 into the open position to provide access to the radiation head.

In Figure 4, when the lock 214 is unlocked, the spring mechanism 216 provides the force necessary to push the cover 208 up and away from the arm 204. That is, the spring provides the force to overcome gravity and therefore cause the cover 208 to move into the open position. This means that the user operating the device does not have to move the cover between the open position and the closed position.

The spring 216 provides a biasing force to open the cover 208 to an optimal open angle alpha α. This can reduce user errors and possible damage to the device since the cover 208 automatically opens to the correct degree.

In some examples the spring 216 instead provides a force less than the force required to overcome gravity. However the biasing force will nonetheless assist the user by providing an amount of biasing force towards the open position, meaning the amount of force required by the user to open the cover is reduced.

When access to the radiation head 202 is no longer required the cover is closed. To close the cover 208 when the device 200 includes a spring 216, the user must push the cover with enough force to overcome the outward bias of the spring, and therefore push the cover back towards the arm and into the closed position.

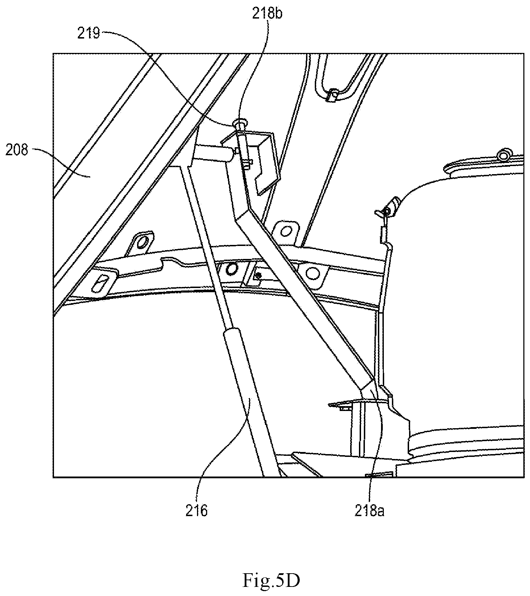

Further, another optional feature is illustrated in the figures. A strut, 218, described in more detail in relation to Figure 5D, is movable between a stowed position and an erect position. The strut is in stowed position when the cover 208 is in the closed position. When the cover is in the open position, the strut is moved into the erect position. In the erect position, the strut is attached at either end to the cover 208 and to the arm 204. The strut therefore 'props' the cover 208 open. The strut 218 takes the weight of the cover 208 when open to maintain it in the open position.

This is advantageous to the user accessing the radiation head 202, since the user does not need to hold the cover open whilst accessing the head. The cover 208 is supported in the open position by the strut. The strut is attached between the cover and the arm so that the cover can be held open even if the gantry or drum 206 is rotated to a different angle of rotation. This might be necessary when performing maintenance on the radiation head. The strut 218 is strong enough to maintain the cover 208 in the open position even whilst the arm 204 rotates.

Specific details of an example of the strut are described in more detail below in relation to Figure 5D.

The strut 218 and the spring mechanism 216 are both optional features. One aspect of the disclosure includes a radiotherapy apparatus with neither the strut not the spring mechanism. In another aspect, the radiotherapy apparatus includes a strut and/or a spring mechanism.

In the aspect which includes both a strut 218 and a spring mechanism 216, the cover 208 opens as follows:
The user unlocks lock 214, such that the far end of the cover 208 and the arm 204 are not connected. The spring mechanism 216 biases the cover 208 into the open position and the cover rotates around hinge 212. Further, the springs limit the open angle. The spring 216 opens the cover to the open angle of 54°. The user moves the strut to the erect position. In the erect position the strut is fixed between the open cover 208 and the arm 204. The strut maintains or locks the cover 208 in the open position, such that the cover 208 does not move relative to the arm 204. The user can then access the radiation head 202. Only one user is needed to open the cover, and the user can perform the operations on the radiation head with both hands, since the cover is held open and the user does not need to hold the cover open.

When access to the radiation head is no longer required, the strut can be moved from the erect position into the stowed position. With the strut 218 in the stowed position the cover can be closed by exerting a force grater than the biasing force into the the closed position. The lock 214 is then locked to fix the cover 208 in the closed position.

Example components of the radiotherapy apparatus are described below in detail. It will be understood that these are specific examples of the components, and that any component suitable for carrying out the function of the component can be used in the radiotherapy apparatus.

Figure 5A shows an example hinge 212. The hinge has a first hinge part 212a which is fixedly attached to the drum 206. The hinge has a second hinge part 212b which is fixedly attached to the cover 208. The first part and the second part are held together by a pin 213. The first part can rotate around the pin relative to the second part. The cover 208 can rotate relative to the drum 206 around the pin. In this way, the cover moves from the closed position and the open position.

In another example, the first hinge part 212a is fixedly attached to the arm 206 and the second hinge part 212b is fixedly attached to the cover 208.

Any hinge mechanism which hingably attaches the cover relative to the arm (i.e. directly to the arm or to a component which is fixably attached relative to the arm) can be used.

In the embodiment in the figures, a single hinge 212 is used to connect the cover to the arm. In another example, two hinges are used. In the example, the two hinges are positioned asymmetrically, such that when the lock 214 is unlocked, the cover 208 initially opens to a small angle, before opening to the full open angle.

Figure 5B shows example brackets 220. The brackets are attached between the cover 208 and the arm 206 and support the movement of the cover between the closed position and the open position. The brackets 220 may further impart an upper limit on the degree to which the cover 208 can open relative to the arm 206. That is, when opening the cover 208 there is an open angle beyond which the cover cannot further rotate away from the arm due to the limitation provided by the brackets.

In one example brackets are positioned between the cover 208 and the arm 206 on both sides of the arm 206. This provides maximum support to the cover 208 between the open and the closed positions. In other examples, there is a bracket 220 attached on only one side of the arm 206. In other examples still, there are no brackets on the device.

The cover 208 is fixed on arm 204 by brackets 220. The brackets 220 provide an attachment point between the arm 204 and the cover. This attachment is stronger than the attachment provided by the hinge 212 and the lock 214. The brackets therefore strengthen the attachment of the cover 208 to the arm 206.

Brackets 220 each include a first part fixedly attached to the arm 206 and a second part fixedly attached to the cover 208. In the closed position the first and second parts align and are secured together by a bolt 221. To move the cover to the open position, the bolt 221 is removed such that the first and second parts of the bracket are no longer attached together. The bolt 221 can be accessed and removed by the user from the exterior of the cover 208. The cover 208 is then free to move away from the arm 206 into the open position.

Figure 5C shows an example lock 214. The lock 214 is includes locking parts 214a and 214b which are integral to, or fixedly attached on, the arm 204 and the cover 208 respectively. The lock 214 has a locked position in which the locking parts 214a and 214b are securely fastened together by fastener 215. Since the locking parts 214a and 214b are positioned on the arm 204 and the cover 208 respectively, when the lock is in the locked position the locking parts, and therefore the arm and the cover, are held together securely. This is the closed position (shown in Figure 2) in which the radiation head 202 is concealed by the cover 208.

In the open position the parts are not securely held together, such that the cover can be moved into the open position as described above. The lock may include a pin 215 to hold together the parts. To unlock the lock, the pin is removed and the parts can be separated. To lock the lock, the pin is inserted between the parts thereby securing them together. Any type of conceivable lock 214 for securely locking the cover to the arm could be used.

Figure 5D shows an example strut 218. The strut 218 has a first end 218a which is hingably mounted to the arm 204. That is, the strut can rotate relative to the arm 204. The strut 218 also has a second end 218B which is configured to attach to a point 219 on the cover 208. In the closed position the strut 218 is not attached at the second end 218b to the cover 208, and is stowed to fit in between the closed cover 208 and the arm 204. When the cover 208 is moved to the open position, the strut 218 can be rotated relative to the arm 204 to meet the cover 208. The second end 218b is attached to the point 219 on the cover 208. The strut 218 holds the cover 208 in the open position.

The strut therefore 'props' the cover 208 open. The strut 218 takes the weight of the cover 208 when open to maintain it in the open position. This is advantageous to the user accessing the radiation head 202, since the user dos not need to hold the cover open whilst accessing the head. The cover 208 is supported in the open position by the strut. The strut is attached between the cover and the arm so that the cover can be held open even if the gantry or drum 206 is rotated to a different angle of rotation. This might be necessary when performing maintenance on the radiation head. The strut 218 is strong enough to maintain the cover 208 in the open position even whilst the arm 204 rotates.

Figures 3 and 4 show the cover in the open position with the gantry at 0° and at 180° respectively. However, it will be understood that the cover 208 can be opened with the gantry at any angle of rotation (i.e. from 0 to 360°). The radiation head may need to be accessed with the gantry at a specific angle - either for maintenance or servicing processes, or the radiation head 202 may need to be accesses is the gantry is stuck at a certain angle.

The radiation head 202 can be accessed with the gantry at any angle of rotation using the same opening mechanism as described above in Figures 3 and 4 with the gantry at 0 and 180° respectively.

The easy open cover in the present disclosure can be opened by a single user with little effort. This reduces man power. A single maintenance engineer can open the cover and freely work on the radiation head whilst the cover is help in the open position. Removing the cover does not require undoing bolts and unscrewing screws, and moving heaving parts, for example a large cover, around. Therefore radiation head can be accessed easily by a single user.

It is quick to access the radiation head with the cover described above. Reducing the time to access the radiation head reduces machine downtime. This reduces cost and increases efficiency.

Finally, the easy open cover provides a safe way to access the radiation head without causing damage to the radiotherapy apparatus. Removing the cover does not require moving heavy parts, and therefore reduces the risk of injury to the user.

Features of the above aspects can be combined in any suitable manner. It will be understood that the above description is of specific embodiments by way of aspect only and that many modifications and alterations will be within the skilled person's reach and are intended to be covered by the scope of the appendant claims.

## Claims

1. A housing system for a radiotherapy apparatus, the system comprising:
a support structure configured to support a source of radiation;
a cover hingeably attached to the support structure and movable between a closed position in which the cover shields the source of radiation and an open position; and
a lock configured to releasably lock the cover in the closed position.

2. A system according to claim 1, wherein the cover is hingably attached to the support structure via a hinge mechanism, wherein the hinge mechanism is positioned at a first end of the cover and the lock is positioned at a second, opposing, end of the cover.

3. The system of claim 1 or claim 2, wherein the support structure comprises:
an arm configured to support the source of radiation; and
a gantry configured to rotate around a rotation axis,
wherein the arm is attached to the gantry.

4. The system of any of claims 1 to 3, further comprising a spring mechanism configured to bias the cover to the open position.

5. The system of claim 4, wherein the spring mechanism has a resting position corresponding to the open position, after which it will not cause any further rotation of the cover.

6. The system of claim 5, wherein, in the open position, the cover is angled relative to the arm at 54°.

7. The system of any of claims 4 to 6, wherein the spring mechanism is an air spring.

8. The system of any preceding claim, further comprising a strut configured to, in an erect position, hold the cover in the open position.

9. The system of any preceding claim further comprising a bracket attached between the cover and the arm, configured to support rotation of the cover between the closed position and the open position.

10. A radiotherapy apparatus comprising:
a housing system according to any of claims 1 to 9; and
a source of radiation supported on the support structure.

11. A method of accessing a source of radiation in the radiotherapy apparatus of claim 10, the method comprising:
with the cover in the closed position, unlocking the lock mechanism; and
rotating the cover around the hinge to the open position.

12. The method of claim 11, wherein the rotation comprises biasing the cover away from the arm into the open position using a spring mechanism.

13. The method of claim 11 or 12, further comprising erecting a strut between the cover and the arm to maintain the cover in the open position.

## Patentansprüche

1. Gehäusesystem für ein Strahlentherapiegerät, wobei das System Folgendes umfasst:
eine Stützstruktur, die zum Stützen einer Strahlungsquelle ausgestaltet ist,
eine Abdeckung, die gelenkig an der Stütztstruktur angebracht und zwischen einer geschlossenen Position, in der die Abdeckung die Strahlungsquelle abschirmt, und einer offenen Position beweglich ist, und
eine Verriegelung, die dazu ausgestaltet ist, die Abdeckung in der geschlossenen Position lösbar zu verriegeln.

2. System nach Anspruch 1, wobei die Abdeckung über einen Scharniermechanismus gelenkig an der Stützstruktur angebracht ist, wobei der Scharniermechanismus an einem ersten Ende der Abdeckung positioniert ist und die Verriegelung an einem zweiten, gegenüberliegenden Ende der Abdeckung positioniert ist.

3. System nach Anspruch 1 oder Anspruch 2, wobei die Stützstruktur Folgendes umfasst:
einen Arm, der zum Stützen der Strahlungsquelle ausgestaltet ist, und
eine zum Drehen um eine Rotationsachse ausgestaltete Gantry,
wobei der Arm an der Gantry angebracht ist.

4. System nach einem der Ansprüche 1 bis 3, ferner umfassend einen Federmechanismus, der zum Vorspannen der Abdeckung in die offene Position ausgestaltet ist.

5. System nach Anspruch 4, wobei der Federmechanismus eine Ruheposition aufweist, die der offenen Position entspricht, wonach er keine weitere Drehung der Abdeckung veranlasst.

6. System nach Anspruch 5, wobei die Abdeckung in der offenen Position um 54° bezüglich des Arms abgewinkelt ist.

7. System nach einem der Ansprüche 4 bis 6, wobei der Federmechanismus eine Luftfeder ist.

8. System nach einem der vorhergehenden Ansprüche, ferner umfassend eine Strebe, die dazu ausgestaltet ist, in einer aufgerichteten Position die Abdeckung in der offenen Position zu halten.

9. System nach einem der vorhergehenden Ansprüche, ferner umfassend eine Halterung, die zwischen der Abdeckung und dem Arm angebracht und zum Stützen der Drehung der Abdeckung zwischen der geschlossenen Position und der offenen Position ausgestaltet ist.

10. Strahlentherapiegerät, umfassend:
ein Gehäusesystem nach einem der Ansprüche 1 bis 9 und eine auf der Stützstruktur gestützte Strahlungsquelle.

11. Verfahren zum Zugriff auf eine Strahlungsquelle in dem Strahlentherapiegerät nach Anspruch 10, wobei das Verfahren Folgendes umfasst:
Entriegeln des Verriegelungsmechanismus, wenn sich die Abdeckung in der geschlossenen Position befindet, und
Drehen der Abdeckung um das Scharnier in die offene Position.

12. Verfahren nach Anspruch 11, wobei die Drehung das Vorspannen der Abdeckung von dem Arm weg in die offene Position unter Verwendung eines Federmechanismus umfasst.

13. Verfahren nach Anspruch 11 oder 12, ferner umfassend das Aufrichten einer Strebe zwischen der Abdeckung und dem Arm, um die Abdeckung in der offenen Position zu halten.

## Revendications

1. Système formant corps pour un appareil de radiothérapie, le système comprenant :
une structure de support conçue pour supporter une source de rayonnements ;
un couvercle attaché par charnière à la structure de support et déplaçable entre une position fermée, dans laquelle le couvercle protège la source de rayonnements, et une position ouverte ; et
un verrou conçu pour verrouiller de manière libérable le couvercle dans la position fermée.

2. Système selon la revendication 1, dans lequel le couvercle est articulé sur la structure de support par le biais d'un mécanisme de charnière, le mécanisme de charnière étant placé à une première extrémité du couvercle et le verrou étant placé à une seconde extrémité opposée du couvercle.

3. Système selon la revendication 1 ou la revendication 2, dans lequel la structure de support comprend :
un support conçu pour supporter la source de rayonnements ; et
un bras conçu pour tourner autour d'un axe de rotation, le support étant attaché au bras.

4. Système selon l'une quelconque des revendications 1 à 3, comprenant, en outre, un mécanisme de ressort conçu pour solliciter le couvercle vers la position ouverte.

5. Système selon la revendication 4, dans lequel le mécanisme de ressort a une position de repos correspondant à la position ouverte, la rotation du couvercle provoquée par celui-ci cessant une fois cette position atteinte.

6. Système selon la revendication 5, dans lequel, dans la position ouverte, le couvercle est placé à un angle de 54° relativement au support.

7. Système selon l'une quelconque des revendications 4 à 6, dans lequel le mécanisme de ressort est un ressort pneumatique.

8. Système selon l'une quelconque des revendications précédentes, comprenant, en outre, une entretoise conçue pour, dans une position déployée, maintenir le couvercle dans la position ouverte.

9. Système selon l'une quelconque des revendications précédentes, comprenant, en outre, une patte de fixation attachée entre le couvercle et le support, conçue pour supporter la rotation du couvercle entre la position fermée et la position ouverte.

10. Appareil de radiothérapie comprenant :
un système formant corps selon l'une quelconque des revendications 1 à 9 ; et
une source de rayonnements supportée sur la structure de support.

11. Procédé pour accéder à une source de rayonnements dans l'appareil de radiothérapie selon la revendication 10, le procédé comprenant :
avec le couvercle dans la position fermée, déverrouiller le mécanisme de verrou ; et
imprimer une rotation au couvercle autour de la charnière jusqu'à la position ouverte.

12. Procédé selon la revendication 11, dans lequel la rotation comprend une sollicitation du couvercle l'éloignant du support et l'amenant à la position ouverte au moyen d'un mécanisme de ressort.

13. Procédé selon la revendication 11 ou 12, comprenant, en outre, le déploiement d'une entretoise entre le couvercle et le support afin de maintenir le couvercle dans la position ouverte.
